(19) Europäisches Patentamt
European Patent Office
Office européen des brevets

(11) Publication number: 0 131 315
A2

(12) # EUROPEAN PATENT APPLICATION

(21) Application number: 84200201.6

(22) Date of filing: 15.02.84

(51) Int. Cl.[4]: A 61 K 9/06
A 61 K 9/08, A 61 K 31/14

(30) Priority: 13.05.83 IT 2109283

(43) Date of publication of application:
16.01.85 Bulletin 85/3

(84) Designated Contracting States:
BE CH DE FR GB IT LI LU NL SE

(71) Applicant: ISTITUTO DE ANGELI S.p.A.
Via Serio, 15
I-20139 Milano(IT)

(72) Inventor: Gallazzi, Alberto
Via Valle Olona 23
I-21052 Busto Arsizio (Varese)(IT)

(72) Inventor: Hammer, Rudolf
Via Fabio Filzi 33
I-20124 Milano(IT)

(72) Inventor: Delucchi, Franco
Via Masaccio 77
I-27058 Voghera (Pavia)(IT)

(72) Inventor: Iamartino, Piero
Via Ampere 11
I-20052 Monza (Milano)(IT)

(72) Inventor: Schiavi, Giovanni Battista
Via Montello 7
I-46041 Asola (Mantova)(IT)

(74) Representative: Appoloni, Romano et al,
ING. BARZANO' & ZANARDO MILANO S.p.A. Via
Borgonuovo 10
I-20121 Milano(IT)

(54) **Nasal preparations comprising anticholinergic quaternary ammonium salts.**

(57) Object of the present invention are pharmaceutical compositions for administration of anticholinergic quaternary ammonium salts containing in the molecule a phenyl group and the nitrogen atom quaternized in a 5- or 6-membered cyclic system. These new pharmaceutical compisitions for nasal administration include solutions, suspensions, creams, ointments and gels.

EP 0 131 315 A2

./...

Croydon Printing Company Ltd.

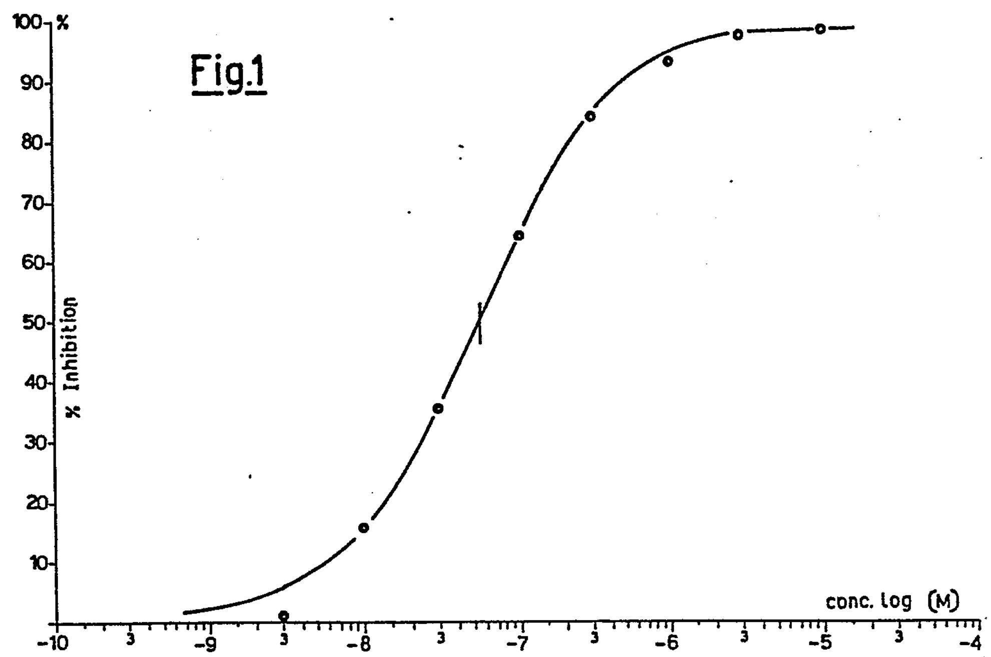

Fig.1
100 %
90
80
70
60
50
40
30
20
10
% Inhibition
conc. log (M)
-10
3
-9
3
-8
3
-7
3
-6
3
-5
3
-4

PHARMACEUTICAL COMPOSITIONS FOR NASAL ADMINISTRATION OF ANTICHOLINERGIC QUATERNARY AMMONIUM SALTS

The present invention relates to the nasal administration of anticholinergic quaternary ammonium salts containing in the molecule a phenyl group and the nitrogen atom quaternized in a 5-or 6- membered cyclic system and to the pharmaceutical compositions containing them.

Among the anticholinergic compounds used in antispasmodic therapy the quaternary ammonium salts containing in the molecule a phenyl group and the nitrogen atom quaternized in a 5-or 6-membered system have the advantage not to penetrate the blood-brain barrier so that the undesired central side-effects are avoided.

It is known that these anticholinergic quaternary ammonium salts are widely used therapeutically exibiting a particularly high spasmolytic activity in spastic conditions of the gastrointestinal tract, of the extrahepatic biliary ducts, of the urogenital and bronchial apparatus.

These salts are also used for instrumental investigations like for example gastroduodenal endoscopy.

Typical representatives of this class of compounds are, for example, the following:

MEPENZOLATE BROMIDE

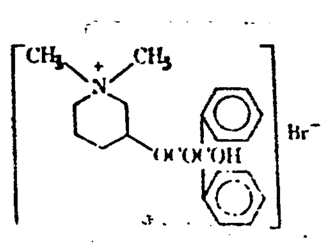

METHSCOPOLAMINE BROMIDE

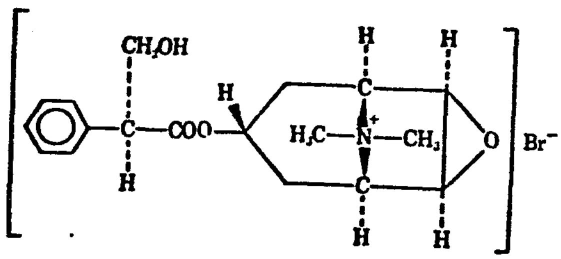

FENPIVERINIUM BROMIDE

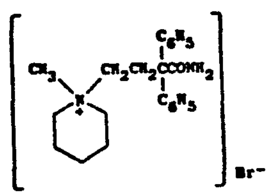

NOVATROPINE

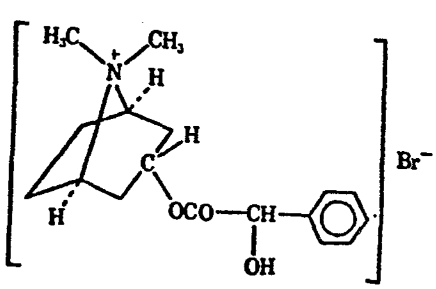

GLYCOPYRROLATE

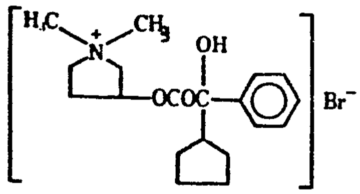

DIPHEMANIL METHYLSULFATE

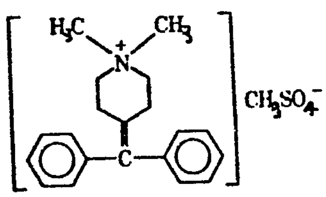

CLIDINIUM BROMIDE

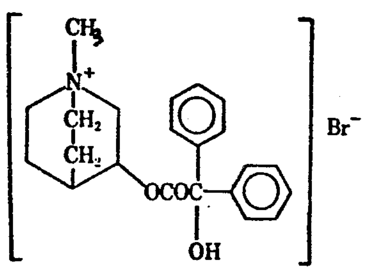

PRIFINIUM BROMIDE

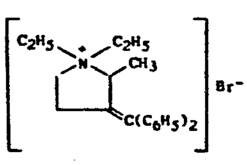

FENTONIUM BROMIDE

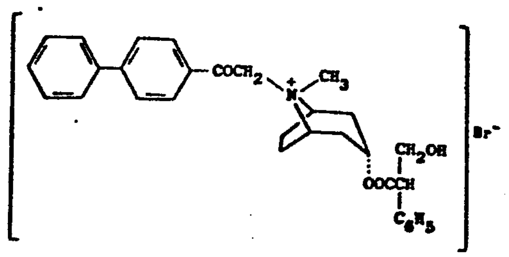

HEXOCYCLIUM METHYLSULFATE

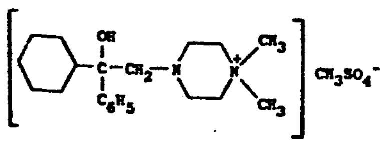

SCOPOLAMINE BUTYLBROMIDE

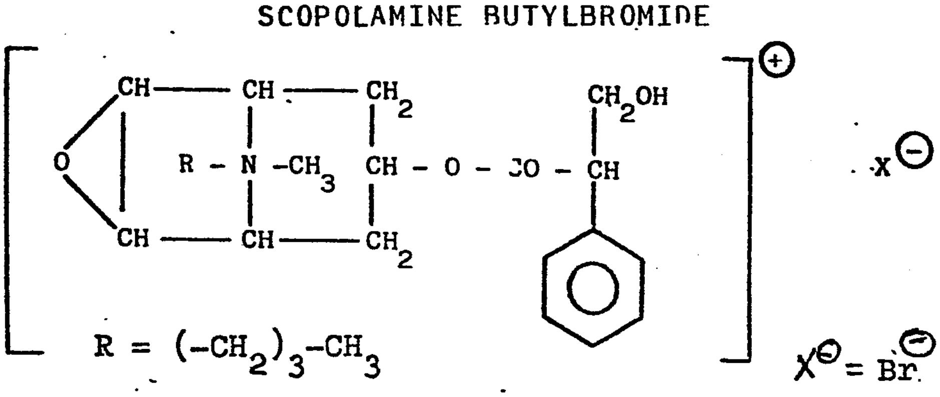

SCOPOLAMINE N-CYCLOPROPYLMETHYL BROMIDE

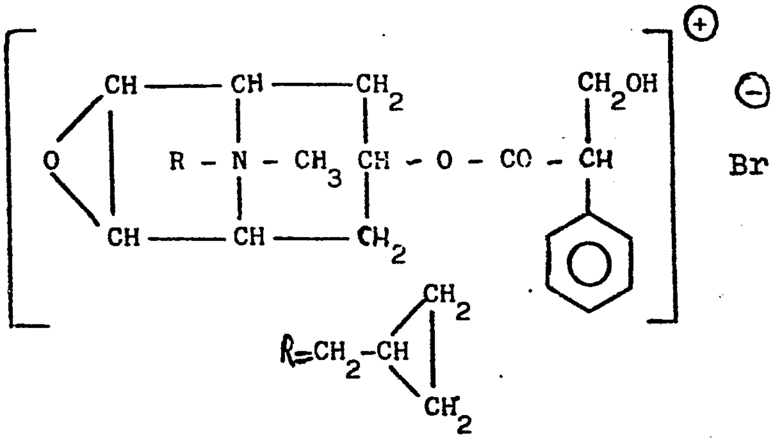

However this class of compounds is poorly and variably absorbed after oral administration. For this reason these compounds are preferably used therapeutically by the intermuscular or intravenous route. The therapeutic limitation of these compounds is evident when the oral administration is essentially the only one feasible route of administration as in the case of the irritable colon.

The poor oral bioavailability of these compounds requests the need of their best absorption. Consequently it is an object of the present invention to provide a new method for administration which provides both a greatly enhanced bioavailability as compared to oral intake and a more convenient administration of these compounds as compared than intravenous, intermuscular or rectal routes. This latter is not feasible in some diseases, for example, in case of the irritable intestine.

This object has been achieved by nasal administration of these compounds advantageously formulated as solution, suspension, cream, ointment, gel, drops or spray adapted for this new administration route. This is proved by the results herein described demonstrating that this new route of administration is suitable for systemic therapy in that it shows a bioavailability about 20 times superior to that of the oral administration.

In fact the urinary excretion of these compounds after nasal administration both in solution and in semisolid

formulation is about 23% of that observed after the intravenous administration. On the other hand, the urinary excretion observed after oral administration achieves only 1.4% of that obtained after the intravenous administration. In addition to the increased bioavailability the new route of administration with adapted pharmaceutical compositions allows a sustained release of these compounds so that a therapy without provoking undesired side-effects is possible.

The present invention provides any pharmaceutical composition acceptable for the nasal administration consisting essentially of (i) anticholinergic quaternary ammonium salts containing in the molecule a phenyl group and the nitrogen atom quaternized in a 5- or 6-membered cyclic system, and (ii) a non toxic pharmaceutically acceptable nasal carrier, in order to obtain after nasal administration an appropriated systemic absorption of the active ingredient.

According to the present invention we have unexpectedly found that these compounds can be administered by nasal route with a bioavailability considerably superior to that obtained after oral administration.

As a representative drug of this class of compounds N-cyclopropylmethylscopolamine bromide was employed in the method and in the compositions according to the present invention. The bioavailability of this compound after administration by nasal and oral route has been examined

and then compared by the following general method. The investigated drug was administered in equal doses by the nasal, oral and intravenous route. 6, 12 and 24 hours after administration the quantitative excretion into the urine for any of the routes of administration was studied. The total amount of the drug excreted during the investigated time intervals was expressed as percentage of the intravenously eliminated amount, considering the latter value as an equivalent for 100% bioavailability.

In detail the following studies have been carried out. For each experiment 10 male Sprague-Dawley rats, weighing 280$\pm$ 20g have been used. For the i.v. administration rats fasted for 18 hours were anaesthetized by intraperitoneal sodium pentobarbital solution 2 ml/kg corresponding to 60 mg/kg/rat. A 4% solution of the drug in buffered saline (pH=7.4) was injected into the femoral vein (50 $\mu$l/rat corresponding to 2 mg of the active ingredient). For the oral administration 18 hours fasted and conscious rats received an 0.2% solution of the drug in saline by means of a stomach tube (1 ml/rat corresponding to 2 mg of the active principle). For the nasal administration the investigated compounds, for example N-cyclopropylmethyl scopolamine bromide, was administered to 18 hours fasted and as above described anaesthetized rats at a dose of 2 mg/rat.

This dose corresponds either to 25 $\mu$l of saline (4% solution) or

5 mg of a 20% gel introduced into both nasal cavities by means of micropipette. After the administration by any route the animals were transfered into metabolic cages and received 3 ml of water by stomach tube. The urine was collected 6,12 and 24 hours after administration and the excreted drug was determined with the following original method which is based on the binding affinity of the drugs to their pharmacological receptors. In fact, it is known that compounds that possess receptor affinity, in this case muscarinic anticholinergics, are able to bind to the receptor proportional to their affinity. It follows from this that knowing the affinity of the drugs it is possible to assay the concentration of the drugs in biological fluids. For this a suitable in vitro receptor preparation and a radio ligand that can bind specifically to the receptor and can be displaced by the test compound is needed. In the present invention tritiated N-methylscopolamine ($^3$H-NMS) in a final concentration of $3.10^{-10}$M that occupies 50% of the muscarinic receptors was employed. As in vitro receptor preparation we used a homogenate from rat cerebral cortex diluted 1:3000 in 20 mM HEPES, 100 mM NaCl and 10 mM $MgCl_2$ (pH 7.4). After incubation of $^3$H-NMS with the receptor preparation receptor bound ligand was separated from free ligand by centrifugation and the amount of the receptor bound ligand was measured as radioactivity (dpm) in the pellet.

In Figure 1 the ability of N-cyclopropylmethylscopolamine

bromide to displace $^3H$-NMS from the muscarinic receptor of cerebral cortex is shown. It is evident that increasing concentrations of N-cyclopropylmethylscopolamine bromide DA-3177 br (abscissa) inhibit the specific binding between $^3H$-NMS and receptor in a dose dependent manner (see fig. 1).

Since the scope of the present invention was to study the bioavailability of quaternary anticholinergics by determining their urinary excretion, it was necessary to demonstrate that the urine per se does not interfere with muscarinic receptor binding. Figure 2 shows that the urine of non treated animals diluted 50- to 500 fold with the above mentioned HEPES-buffer does not influence the binding of the radioligand with the receptor (see fig. 2).

Therefore, on the basis of the standard curve (Fig. 1) and having clarified that the urine per se does not interfere with muscarinic receptor binding (Fig. 2) it is possible to determine the amount of quaternary anticholinegrics excreted into the urine.

The following tables report the urinary excretion of N-cyclopropylmethylscopolamine bromide in each investigated rat and for the considered different routes of administrations.

TABLE 1: Intravenous administration of N-cyclopropyl-methylscopolamine bromide in the rat.
Reported are the amounts of the coumpound found in the urine 6,12 and 24 hours after administration.
The values are expressed as percentage of the total administered dose.

| rat. n. | hours 0-6 | 6-12 | 12-24 | 0-24 |
|---|---|---|---|---|
| 1 | 26.39 | 3.20 | 0.72 | 30.34 |
| 2 | 27.82 | 0.86 | 0.00 | 28.68 |
| 3 | 13.96 | 1.75 | 0.69 | 16.40 |
| 4 | 17.81 | 2.36 | 1.94 | 22.11 |
| 5 | 20.41 | 2.66 | 1.89 | 24.96 |
| 6 | 22.93 | 2.78 | 0.35 | 26.06 |
| 7 | 23.18 | 4.32 | 3.28 | 30.78 |
| 8 | 26.80 | 1.21 | 0.20 | 28.21 |
| 9 | 24.46 | 2.00 | 0.27 | 26.73 |
| 10 | 29.68 | 1.19 | 0.10 | 30.97 |
| mean | 23.24($\pm$4.8) | 2.24($\pm$1.05) | 0.94($\pm$1.07) | 26.52($\pm$4.5) |

TABLE 2: Oral administration of N-cyclopropylmethylscopolamine bromide in the rat. Reported are the amounts of the compound found in the urine 6,12 and 24 hours after administration.

The values are expressed as percentage of the total administered dose.

| rat. n. hours | 0-6 | 6-12 | 12-24 | 0-24 |
|---|---|---|---|---|
| 1 | 0.37 | 0.10 | 0.02 | 0.49 |
| 2 | 0.37 | 0.11 | 0.00 | 0.48 |
| 3 | 0.29 | 0.07 | 0.03 | 0.39 |
| 4 | 0.31 | 0.06 | 0.03 | 0.40 |
| 5 | 0.29 | 0.06 | 0.02 | 0.37 |
| 6 | 0.30 | 0.00 | 0.00 | 0.30 |
| 7 | 0.33 | 0.06 | 0.03 | 0.42 |
| 8 | 0.14 | 0.04 | 0.04 | 0.22 |
| 9 | 0.15 | 0.27 | 0.02 | 0.44 |
| 10 | 0.13 | 0.04 | 0.01 | 0.18 |
| mean | 0.27 (±0.09) | 0.08 (±0.07) | 0.02 (±0.01) | 0.37 (±0.10) |

TABLE 3: Nasal administration of N-cyclopropylmethylscopolamine bromide in solution in the rat.

Reported are the amounts of the compound found in the urine 6,12 and 24 hours after administration. The value are expressed as percentage of the total administered dose.

| rat. n. | hours 0-6 | 6-12 | 12-24 | 0-24 |
|---|---|---|---|---|
| 1 | 6.25 | 0.25 | 0.09 | 6.59 |
| 2 | 5.53 | 0.13 | 0.09 | 5.75 |
| 3 | 8.86 | 0.23 | 0.14 | 9.23 |
| 4 | 2.93 | 0.19 | 0.05 | 3.17 |
| 5 | 2.68 | 0.39 | 0.04 | 3.11 |
| 6 | 4.82 | 0.65 | 0.08 | 5.55 |
| 7 | 11.42 | 0.28 | 0.18 | 11.88 |
| 8 | 5.17 | 0.86 | 0.09 | 6.12 |
| 9 | 4.55 | 0.44 | 0.08 | 5.07 |
| 10 | 4.12 | 0.33 | 0.07 | 4.52 |
| mean | 5.63(±2.68) | 0.38(±0.22) | 0.09 (±0.04) | 6.10(±0.2) |

TABLE 4: Nasal administration of N-cyclopropylmethylscopolamine bromide as a gel in the rat.

Reported are the amounts of the compound found in the urine 6,12 and 24 hours after administration.

The value are expressed as percentage of the total administered dose.

| rat n. | hours 0-6 | 6-12 | 12-24 | 0-24 |
|---|---|---|---|---|
| 1 | 4.71 | 4.58 | 1.9 | 11.19 |
| 2 | 1.65 | 1.63 | 2.0 | 5.28 |
| 3 | 2.39 | 2.17 | 0.63 | 5.19 |
| 4 | 3.12 | 3.24 | 2.04 | 8.40 |
| 5 | 2.86 | 2.90 | 1.18 | 6.94 |
| 6 | 1.47 | 1.53 | 0.05 | 3.05 |
| 7 | 1.08 | 1.00 | 0.74 | 2.82 |
| 8 | 2.31 | 2.18 | 2.37 | 6.86 |
| 9 | 2.20 | 2.35 | 1.04 | 5.59 |
| 10 | 2.07 | 2.00 | 1.19 | 5.26 |
| mean | 2.39 (±0.05) | 2.36(±1.01) | 1.31-±0.74) | 6.06 (+2.47) |

On the basis of the values reported in Tables 1-4 relative and absolute bioavailability for the various routes of administration and formulations can be estimated (Table 5).

TABLE 5: Comparison of the bioavailability of N-cyclopropylmethylscopolamine bromide after oral administration and after the nasal administration of the compound in solution and gel.

PERCENT URINARY EXCRETION BY VARIOUS ROUTES OF ADMINISTRATION

| | NASAL | | | |
|---|---|---|---|---|
| HOURS | GEL | SOLUTION | OS | I.V. |
| 0-6 | 2.39 | 5.63 | 0.27 | 23.34 |
| 6-12 | 2.36 | 0.38 | 0.08 | 2.24 |
| 12-24 | 1.31 | 0.09 | 0.02 | 0.94 |
| 0-24 | 6.06 | 6.10 | 0.37 | 26.52 |

PERCENT URINARY EXCRETION BY OTHER ROUTES RELATIVE TO I.V. ADMINISTRATION

| | | | | |
|---|---|---|---|---|
| 0-6 | 9.01 | 21.23 | 1.02 | 88.01 |
| 6-12 | 8.90 | 1.43 | 0.30 | 8.45 |
| 12-24 | 4.94 | 0.34 | 0.08 | 3.54 |
| 0-24 | 22.85 | 23.00 | 1.40 | 100.00 |

It is evident from Table 5 that the administration by the nasal route, with both galenic formulations, solution and gel leads to an absolute bioavailability of 23%, which contrasts clearly the poor bioavailability of 1.4% after oral administration. Thus, the absorption of N-cyclopropylmethylscopolamine bromide after nasal

administration both by solution and in the form of gel is 16-times greater as compared to the per oral form. As can also be seen from Table 5 the bioavailability of N-cyclopropylmethylscopolamine bromide after administration in solution by nasal route follows the same profile (of those) observed after administration of the compound both by oral and intravenous routes.
In fact the profile of the urinary excretion of the compound after all these administrations shows a maximum between 0-6 hours, decreases strongly between 6-12 hours showing a minimum between 12-24 hours.
On the contrary the bioavailability of N-cyclopropylmethylscopolamine bromide after administration as a gel still by nasal route follows a profile quite different from those previously observed.
In fact, as can be seen from Table 5, the urinary excretion after this administration by nasal route results nearly constant in both the intervals, between 0-6 hours and between 6-12 hours only decreases between 12-24 hours.
These results clearly show that prolonged release of N-cyclopropylmethylscopolamine bromide can be achieved via nasal administration in suitable formulations.
Any of the selected drugs intended for therapeutic use according to the present invention can be administered nasally to warm-blooded animals conveniently by pharmaceutical formulation acceptable to nasal dosage form comprising the desired drug in a therapeutically effective amount (i.e. depending on the selected drug, on the

effective amount of the selected drug suitable for antispasmodic activity etc.) together with a non toxic pharmaceutically acceptable nasal carrier therefor. Suitable non toxic pharmaceutically acceptable nasal carriers will be apparent to those skilled in the art of nase pharmaceutical formulations. For those not skilled in the art, reference is made to the text entitled: "REMINGTON'S PHARMACEUTICAL SCIENCES", 16th edition, 1980. Obviously the choice of suitable carriers will depend on the exact nature of the particular nasal dosage form desired, e.g., whether the quaternary ammonium salt is to be formulated into a nasal solution (for use as drops or as a spray), a nasal suspension, a nasal ointment or a nasal gel. Preferred nasal dosage forms are solutions, creams and gels which contain a major amount of water in addition to the active ingredient.
Minor amounts of other ingredients such as preservatives, buffering agents, wetting agents and jelling agents may also be present.

This type of composition can be used in the treatment of all different therapeutic indications in which the selected drug is effective also after the other routes of administration.

Obviously the therapeutic dosage range for nasal administration of the anticholinergic quaternary ammonium salts according to the present invention will vary with the size of the patient, the condition for which the

drugs are administered, and of course, with the activity of any of the selected drugs intended for use.

A typical dose of N-cyclopropylmethylscopolamine bromide would be 5 to 50 mg administered nasally three times daily. The doses of the other selected drugs intended for use in the present invention would be 1 to 200 mg identically administered.

The quantity of nasal dosage form needed to deliver the desired dose will of course depend on the concentration of drug in the composition.

The volume of solution or gel which would be needed e.g. to deliver N-cyclopropylmethylscopolamine bromide in the doses specified above would be 0.05 to 0.5 ml of 10% solution or 0.05 to 0.5 g of cream and 0.025 to 0.25 g of 20% gel or ointment.

Examples of typical nasal pharmaceutical compositions containing N-cyclopropylmethylscopolamine bromide are set forth below. However it is to be understood that these compositions are given by way of illustration only and are not to be considered as limiting the invention either in spirit or in scope as many modifications both in materials and in methods will be apparent to those skilled in the art.

EXAMPLE 1

10 grams of N-cyclopropylmethylscopolamine bromide are dissolved in 80 ml of distilled water at 50°C. A quantity of water sufficient to bring the total volume to 100 ml is then added to adjust the solution at R.T.; the solution

is then sterilized by being passed through a 0.2 micron Millipore filter.

The final composition of the solution is:

| | | |
|---|---|---|
| N-cyclopropylmethylscopolamine bromide | | 10 g |
| Water, purified | q.s. | 100 ml |

EXAMPLE 2

20 grams of N-cyclopropylmethylscopolamine bromide are dissolved at 80°C in 53 g of distilled water.

18 g of Cethylstearyl alcohol and 9 g of a Polyol ester of fatty acids are melted at 80°C, with stirring, in a suitable apparatus.

The previous prepared solution is added to this melted mixture, with stirring, at 80°C.

The resultant homogeneous mixture is allowed to reach, with stirring, the room temperature.

The final composition of the sustained release gel is:

| | |
|---|---|
| N-cyclopropylmethylscopolamine bromide | 20 g |
| Cetylstearyl alcohol | 18 g |
| Polyol ester of fatty acids | 9 g |
| Water, purified | 53 g |

EXAMPLE 3

10 g of N-cyclopropylmethylscopolamine bromide are dissolved at 70°C in 70 g of distilled water.

20 g of a Polyglycolic ester of fatty acids are melted at 65° C in a suitable container with stirring.

The solution previously prepared is added to this melted compound with stirring still at 65°C.

The resultant homogeneous mixture is allowed to reach, with stirring, the room temperature.

The final composition of the cream is:

| | |
|---|---|
| N-cyclopropylmethylscopolamine bromide | 10 g |
| Polyglycolic ester of fatty acids | 20 g |
| Water purified | 70 g |

EXAMPLE 4

80 g of Vaseline are melted in a suitable container at 65°C. 20 g of N-cyclopropylmethylscopolamine, finely sieved, are dispersed in this melted vaseline, with gentle stirring at 65°C.

The homogeneous mixture is allowed under stirring and without heating to reach about 50°C.

The mixture so obtained is allowed to stand, without stirring and let to reach the room temperature.

The final composition of the ointment is:

| | |
|---|---|
| N-cyclopropylmethylscopolamine bromide | 20 g |
| Vaseline | 80 g |

CLAIMS:

1. A pharmaceutically acceptable nasal composition, to obtain for nasal administraiton a systemic therapeutic anticholinergic response in a warm-blooded animal, consisting essentially of, for nasal dosage unit, (i) a systemically therapeutically effective anticholinergic amount of quaternary ammonium salts containing in the molecule a phenyl group and the nitrogen atom quaternized in a 5- or 6-membered cyclic system, and (ii) a non toxic pharmaceutically acceptable nasal carrier therefor.

2. A composition as defined in claim 1, said composition comprising a nasal solution.

3. A composition as defined in claim 1, said composition comprising a nasal suspension.

4. A composition as defined in claim 1, said composition comprising a nasal ointment.

5. A composition as defined in claim 1, said composition comprising a nasal cream.

6. A composition as defined in claim 1, said composition comprising a nasal gel.

7. A composition as defined in claim 1, said composition comprising a sustained release nasal gel.

8. A composition as defined in claim 1, said composition comprising nasal drops.

9. A composition as defined in claim 1, said composition comprising a nasal spray.

10. A pharmaceutically acceptable nasal composition,

in dosage unit form to obtain for nasal administration a systemic therapeutic anticholinergic response in a warm-blooded animal, consisting essentially of, per nasal dosage unit, (i) a systematically therapeutically effective unit anticholinergic amount of quaternary ammonium salts containingin the molecule a phenyl group and the nitrogen atom quaternized in a 5- or 6-membered cyclic system, and (ii) a non toxic pharmaceutically acceptable nasal carrier therefor.

11. A composition as defined in claim 10, said composition comprising a nasal solution, a nasal suspension, a nasal ointment, a nasal cream or a nasal gel.

12. A composition as claimed in claim 11, in which the nasal gel is a sustained release formulation.

13. A composition as defined in claim 1 in which (i) is a systematically therapeutically effective anticholinergic amount of N-cyclopropylmethylscopolamine bromide.

14. A composition as defined in claim 10, 11 or 12 in which (i) is a systematically therapeutically effective unit anticholinergic amount of N-cyclopropylmethylscopolamine bromide.

15. A pharmaceutically acceptable nasal composition as claimed in claim 1, 10 for use in a method for inducing therapeutic levels of N-cyclopropylmethylscopolamine bromide in a warm-blooded animal.

Fig.1

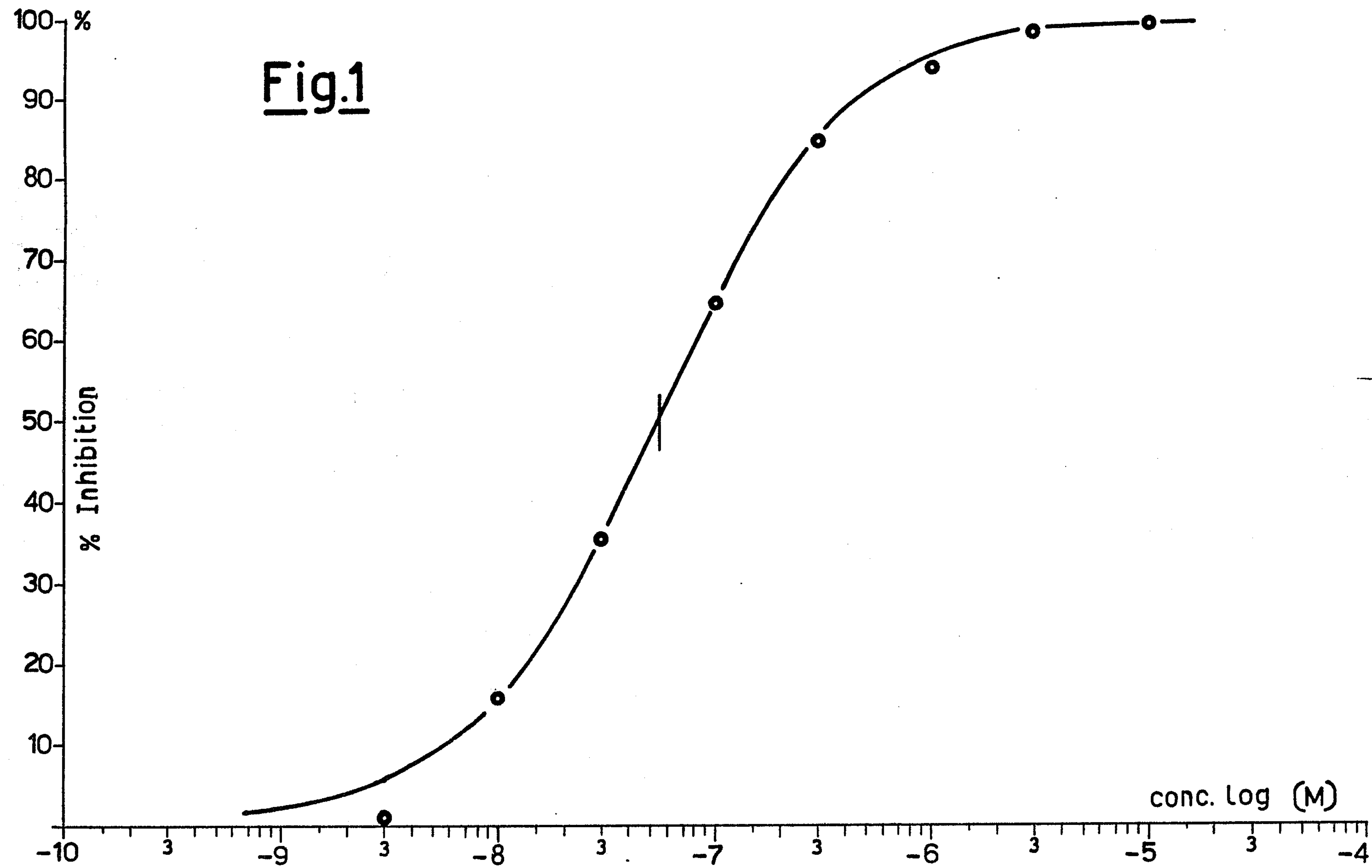

100 %
90
80
70
60
50
40
30
20
10
% Inhibition
conc. Log (M)
−10
3
−9
3
−8
3
−7
3
−6
3
−5
3
−4

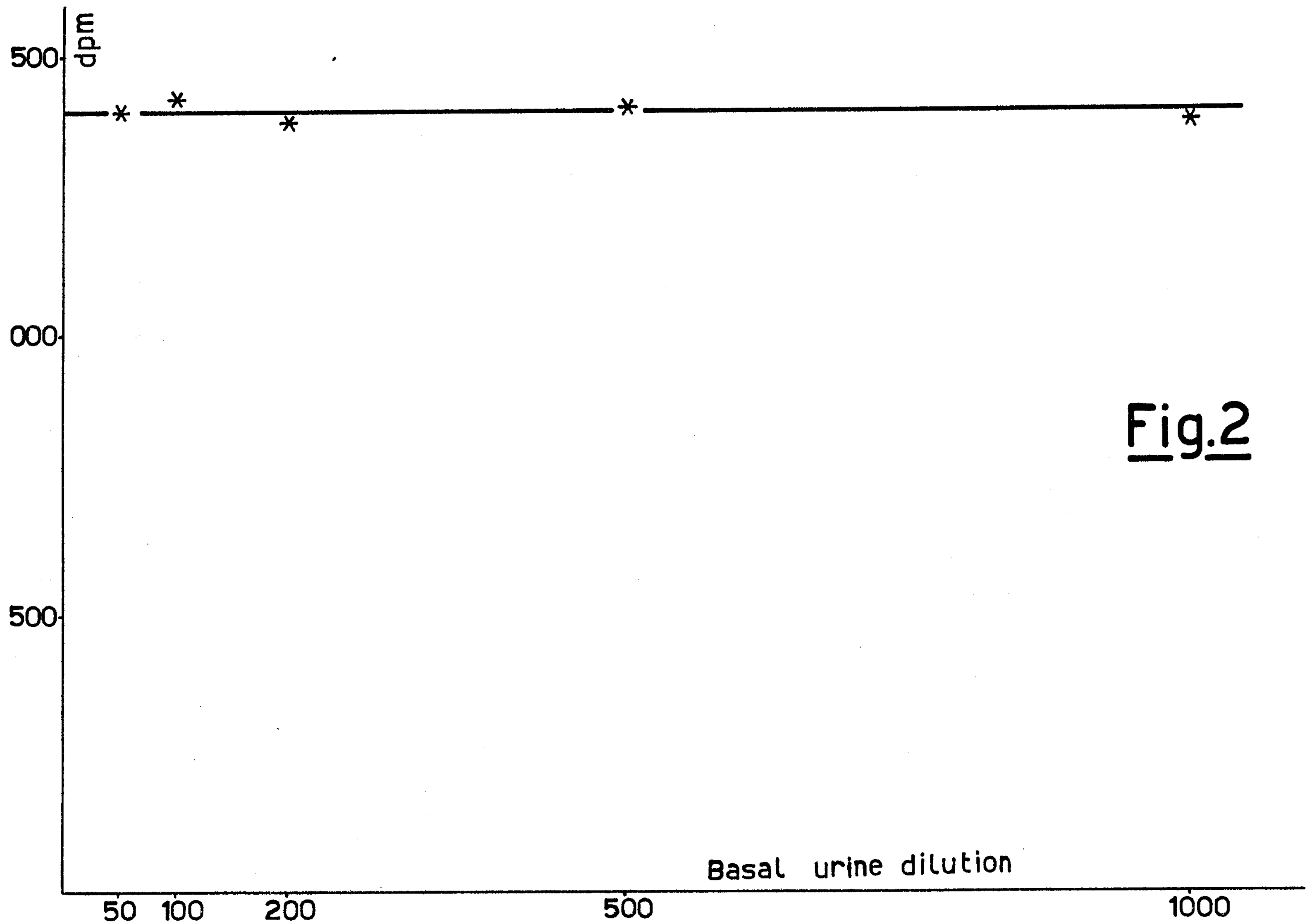


Fig.2